# EUROPÄISCHE PATENTSCHRIFT

(11) **EP 3 191 077 B1**
(45) Veröffentlichungstag und Bekanntmachung des Hinweises auf die Patenterteilung: **26.12.2018**
(21) Anmeldenummer: 15756587.0
(22) Anmeldetag: 18.08.2015
(51) Int. Cl.: A61K 8/58, A61Q 5/06, A61Q 5/10, A61K 8/891, A61K 8/90

(54) **FÄRBEMITTEL MIT PROTEIN-SILOXAN-POLYMEREN**
OXIDATION COLOURING COMPOSITION WITH PROTEIN-SILOXANE POLYMERS
COLORANT D'OXYDATION CONTENANT DES POLYMÈRES PROTÉINE-SILOXANE

(30) Priorität: 09.09.2014 DE 102014218005
(43) Veröffentlichungstag der Anmeldung: 19.07.2017
(73) Patentinhaber: Henkel AG & Co. KGaA, 40589 Düsseldorf (DE)
(72) Erfinder: KERL, Sylvia, 22763 Hamburg (DE); GROSJACQUES, Camille, 20255 Hamburg (DE)
(86) Internationale Anmeldenummer: PCT/EP2015/068879
(87) Internationale Veröffentlichungsnummer: WO 2016/037806

(56) Entgegenhaltungen:
- WO-A1-2014/143667
- JP-A- 2001 151 643
- JP-A- 2002 265 339
- JP-A- 2004 075 630
- US-A- 5 412 074
- US-A1- 2003 235 554
- US-A1- 2013 156 716
- DOERING THOMAS ET AL: "Super mild oxidation coloring: preventing hair damage at the molecular level", IFSCC MAGAZINE, ALLURED PUB., CAROL STREAM, IL, US, Bd. 10, Nr. 4, 1. Januar 2007 (2007-01-01) , Seiten 323-329, XP009121677, ISSN: 1520-4561
- DATABASE GNPD [Online] MINTEL; September 2009 (2009-09), "Root Rehab Emergency Retouch Kit", XP002745115, Database accession no. 1186528

## Beschreibung

Die vorliegende Erfindung betrifft kosmetische Mittel zur Färbung keratinischer Fasern, welche Protein-Siloxan-Polymere enthalten.

Weiterhin betrifft die vorliegende Erfindung eine Verpackungseinheit (Kit-of-parts), enthaltend ein erfindungsgemäßes kosmetisches Mittel sowie eine Oxidationsmittelzubereitung.

Darüber hinaus betrifft die vorliegende Erfindung ein Verfahren zum Färben von keratinischen Fasern unter Verwendung eines erfindungsgemäßen kosmetischen Mittels sowie einer Oxidationsmittelzubereitung.

Zudem betrifft die vorliegende Erfindung die Verwendung eines erfindungsgemäßen kosmetischen Mittels zur Erhöhung der Pflege keratinischer Fasern.

Schließlich betrifft die vorliegende Erfindung die Verwendung einer erfindungsgemäßen Verpackungseinheit zur Herstellung eines kosmetischen Mittels zur Farbveränderung keratinischer Fasern mit erhöhter Pflege der keratinischen Fasern.

Die Veränderung der Form und der Farbe von Haaren stellt einen wichtigen Bereich der modernen Kosmetik dar. Dadurch kann das Erscheinungsbild der Haare sowohl an aktuelle Modeströmungen als auch an die individuellen Wünsche des einzelnen Verbrauchers angepasst werden. Die modische Farbgestaltung von Frisuren oder die Kaschierung von ergrautem oder weißem Haar mit modischen oder natürlichen Farbtönen erfolgt üblicherweise mit farbverändernden Mitteln. Diese Mittel sollen neben einer hohen Färbeleistung zusätzliche Eigenschaften, wie beispielsweise die Steigerung des Haarvolumens, aufweisen.

Zur Bereitstellung farbverändernder kosmetischer Mittel, insbesondere für die Haut oder für keratinhaltige Fasern, wie beispielsweise menschliche Haare, sind im Stand der Technik verschiedene Färbesysteme bekannt.

Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss jedoch üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Für temporäre Färbungen werden üblicherweise Färbe- oder Tönungsmittel verwendet, welche als färbende Komponente sogenannte Direktzieher enthalten. Hierbei handelt es sich um Farbstoffmoleküle, welche direkt auf das Substrat aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als oxidativen Färbungen, so dass sehr viel früher eine vielfach unerwünschte Nuancenverschiebung oder ein sichtbarer homogener Farbverlust eintritt.

Schließlich hat ein weiteres Färbeverfahren große Beachtung gefunden. Bei diesem Verfahren werden Vorstufen des natürlichen Haarfarbstoffes Melanin auf das Substrat, z.B. Haare, aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Insbesondere bei mehrfacher Anwendung von Mitteln mit 5,6-Dihydroxyindolin ist es möglich, bei Personen mit ergrauten Haaren die natürliche Haarfarbe wiederherzustellen. Die Ausfärbung kann dabei mit Luftsauerstoff als einzigem Oxidationsmittel erfolgen, so dass auf weitere Oxidationsmittel verzichtet werden kann. Bei Personen mit ursprünglich mittelblondem bis braunem Haar kann das 5,6-Dihydroxyindolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Im Dokument "Super mild oxidation coloring: preventing hair damage at the molecular level" (IFSCC MAGAZINE, Nr. 4, 1. Januar 2007, Seiten 323-329, ISSN: 1520-4561) wird ein Färbemittel offenbart, welches ein Protein-Siloxan-Polymer in Form Cystine-bis-PG propyl silantriol sowie Oxidationsfarbstoffprodukte enthält. Es wird mit einer Oxidationsmittelzusammensetzung vermischt.

Die im Stand der Technik bekannten Färbemittel führen jedoch nicht immer zu der gewünschten hohen Färbeleistung oder weisen zusätzliche gewünschte Eigenschaften, wie beispielsweise eine verbesserte Pflegewirkung, auf.

Der vorliegenden Erfindung lag daher die Aufgabe zugrunde, ein kosmetisches Mittel zur Färbung keratinischer Fasern bereitzustellen, welches die Nachteile des Standes der Technik vermeidet bzw. zumindest abschwächt und welches in einer verbesserten Pflege der mit diesen Mitteln gefärbten keratinischen Fasern resultiert.

Es wurde nun überraschenderweise gefunden, dass der Zusatz von mindestens einem Protein-Siloxan-Polymer in kosmetischen Mitteln zur Färbung keratinischer Fasern in einer verbesserten Pflegewirkung resultiert, ohne die Färbeleistung der erfindungsgemäßen kosmetischen Mittel oder deren Lagerstabilität negativ zu beeinflussen.

Der Gegenstand der Erfindung ist daher ein Verpackungseinheit (Kit of Parts), umfassend -getrennt voneinander konfektioniert -
(a) mindestein einen Container (C1), enthaltend ein kosmetisches Mittel zur Farbveränderung keratinischer Fasern, umfassend in einem kosmetisch verträglichen Träger
   a) mindestens eine Verbindung, ausgewählt aus der Gruppe von Oxidationsfarbstoffvorprodukten sowie deren Mischungen,
   b) mindestens ein Protein-Siloxan-Polymer, in einer Gesamtmenge von 0,02 bis 0,1 Gew.-%, bezogen auf das Gesamtgewicht des kosmetischen Mittels, enthaltend mindestens eine Struktureinheit der Formel (I) und/oder mindestens eine Struktureinheit der Formel (II) 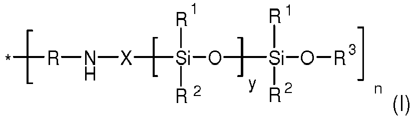 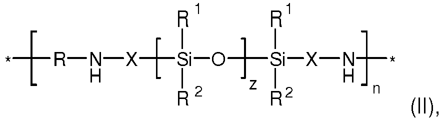 worin
      R für ein aus der Kartoffel isoliertes Proteinhydrolysat mit mindestens einer NH₂-Gruppe steht,
      X für eine Gruppe *-CH₂-CH(OH)-CH₂-O-(CH₂)₃-* steht,
      R¹ und R², jeweils unabhängig voneinander, für eine Hydroxylgruppe stehen,
      R³ für ein Wasserstoffatom steht,
      n für ganze Zahlen von 1 bis 101 steht,
      y für ganze Zahlen von 0 bis 1.000, vorzugsweise von 0 bis 500, insbesondere von 0 bis 100, steht, und
      z für ganze Zahlen von 1 bis 1.000, vorzugsweise von 1 bis 500, insbesondere von 1 bis 100, steht, wobei der hydrolysierte Proteinrest R in mittleres Molekulargewicht Mw von 295 Da bis 26.000 Da aufweist,
(b) mindestens einen Container (C2), enthaltend eine Oxidationsmittelzubereitung, welche in einem kosmetisch verträglichen Träger mindestens ein Oxidationsmittel in einer Gesamtmenge von 0,5 bis 7,0 Gew.-%, bezogen auf das Gesamtgewicht der Oxidationsmittelzubereitung, sowie mindestens eine Säure enthält.

Unter dem Begriff "keratinische Fasern oder auch Keratinfasern" sind erfindungsgemäß Pelze, Wolle, Federn sowie menschliche Haare zu verstehen. Im Rahmen der vorliegenden Erfindung ist es besonders bevorzugt, wenn die kosmetischen Mittel zur Färbung von menschlichen Haaren verwendet werden.

Weiterhin wird unter dem Begriff "Kämmbarkeit" im Rahmen der vorliegenden Erfindung sowohl die Kämmbarkeit der nassen Faser, als auch die Kämmbarkeit der trockenen Faser verstanden.

Zudem werden unter dem Begriff "Protein-Siloxan-Polymere" erfindungsgemäß Polymere verstanden, welche sowohl Proteinreste als auch Siloxaneinheiten enthalten und welche durch Reaktion eines Proteins mit einem organofunktionellen Siloxan erhältlich sind. Unter Proteinrest werden in diesem Zusammenhang chemische Verbindungen verstanden, welche durch Peptid-Bindungen Säureamid-artig verknüpfte Kondensationsprodukte von Aminosäuren darstellen und über einen Spacer X an das Siloxanrückrat gebunden sind. Die Anzahl der Aminosäuren in den Proteinresten beträgt bevorzugt mindestens 5 und maximal 1.000 Aminosäuren. Unter dem Begriff "Proteinrest" sind erfindungsgemäß auch Hydrolysate eines Proteins zu verstehen, welche Proteinfraktionen mit verschiedenen Aminosäuresequenzen und Molekulargewichten enthalten. Die Protein-Siloxan-Polymere können linear oder vernetzt sein. Vernetzte Polymere im Rahmen der Erfindung liegen insbesondere vor, wenn die Gruppen R¹ und/oder R² für einen Rest der Formel (IIIb) stehen.

Darüber hinaus werden unter dem Begriff "Fettalkohole" im Rahmen der vorliegenden Erfindung aliphatische, langkettige, einwertige, primäre Alkohole verstanden, welche unverzweigte Kohlenwasserstoffreste mit 6 bis 30 Kohlenstoffatomen aufweisen. Die Kohlenwasserstoffreste können gesättigt aber auch ein- oder mehrfach ungesättigt sein.

Schließlich werden unter dem Begriff "Fettsäuren" im Rahmen der vorliegenden Erfindung aliphatische Monocarbonsäuren mit unverzweigter Kohlenstoffkette verstanden, welche Kohlenwasserstoffreste mit 6 bis 30 Kohlenstoffatomen aufweisen. Die Kohlenwasserstoffreste können entweder gesättigt oder auch ein- oder mehrfach ungesättigt sein.

Die Angabe der Gesamtmenge in Bezug auf die Komponenten des kosmetischen Mittels bezieht sich vorliegend - sofern nichts anderes angegeben ist - auf die Gesamtmenge an Aktivsubstanz der jeweiligen Komponente. Weiterhin bezieht sich die Angabe der Gesamtmenge in Bezug auf die Komponenten des kosmetischen Mittels - sofern nichts anderes angegeben ist - auf das Gesamtgewicht des oxidationsmittelfreien erfindungsgemäßen kosmetischen Mittels.

Die erfindungsgemäßen kosmetischen Mittel enthalten einen kosmetischen Träger. Erfindungsgemäß bevorzugt ist der kosmetische Träger wässrig, alkoholisch oder wässrig-alkoholisch. Im Rahmen der vorliegenden Erfindung können beispielsweise Cremes, Emulsionen, Gele oder tensidhaltige schäumende Lösungen, wie beispielsweise Shampoos, Schaumaerosole oder andere Zubereitungen, welche für die Anwendung auf dem Haar geeignet sind, eingesetzt werden.

Ein wässriger Träger enthält im Sinne der Erfindung mindestens 30 Gew.-%, insbesondere mindestens 50 Gew.-% Wasser, bezogen auf das Gesamtgewicht des kosmetischen Mittels.

Unter wässrig-alkoholischen Trägern sind im Sinne der vorliegenden Erfindung wasserhaltige Zusammensetzungen, enthaltend einen C₁-C₄-Alkohol in einer Gesamtmenge von 3 bis 90 Gew.-%, bezogen auf das Gesamtgewicht des kosmetischen Mittels, insbesondere Ethanol bzw. Isopropanol, zu verstehen.

Die erfindungsgemäßen kosmetischen Mittel können zusätzlich weitere organische Lösungsmittel, wie beispielsweise Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel, wobei das Lösungsmittel in einer Gesamtmenge von 0,1 bis 30 Gew.-%, vorzugsweise von 1 bis 20 Gew.-%, insbesondere von 2 bis 10 Gew.-%, bezogen auf das Gesamtgewicht des kosmetischen Mittels, enthalten ist.

Das erfindungsgemäße kosmetische Mittel enthält als ersten wesentlichen Bestandteil a) eine Verbindung, ausgewählt aus der Gruppe von Oxidationsfarbstoffvorprodukten (OFV).

In einer bevorzugten Ausführungsform enthalten erfindungsgemäße kosmetische Mittel mindestens ein Oxidationsfarbstoffvorprodukt.

Oxidationsfarbstoffvorprodukte können aufgrund ihres Reaktionsverhaltens in zwei Kategorien eingeteilt werden, sogenannte Entwicklerkomponenten und Kupplerkomponenten. Entwicklerkomponenten können mit sich selbst den eigentlichen Farbstoff ausbilden. Sie können daher als alleinige Verbindungen im erfindungsgemäßen kosmetischen Mittel enthalten sein. In einer bevorzugten Ausführungsform enthalten die erfindungsgemäßen kosmetischen Mittel daher mindestens ein Oxidationsfarbstoffvorprodukt vom Entwicklertyp. Es kann im Rahmen der vorliegenden Erfindung jedoch auch vorgesehen sein, dass die erfindungsgemäßen kosmetischen Mittel mindestens ein Oxidationsfarbstoffvorprodukt vom Kupplertyp enthalten. Besonders gute Ergebnisse in Bezug auf die Färbung keratinischer Fasern werden erhalten, wenn die erfindungsgemäßen kosmetischen Mittel mindestens ein Oxidationsfarbstoffvorprodukt vom Entwicklertyp und mindestens ein Oxidationsfarbstoffvorprodukt vom Kupplertyp enthalten.

Die Entwickler- und Kupplerkomponenten werden üblicherweise in freier Form eingesetzt. Bei Substanzen mit Aminogruppen kann es jedoch bevorzugt sein, deren Salzform, insbesondere in Form der Hydrochloride und Hydrobromide oder der Sulfate, einzusetzen.

Erfindungsgemäß sind kosmetische Mittel bevorzugt, welche die Entwickler- und/oder Kupplerkomponenten jeweils in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthalten.

In einer weiteren bevorzugten Ausführungsform ist das erfindungsgemäße kosmetische Mittel daher dadurch gekennzeichnet, dass es ein Oxidationsfarbstoffvorprodukt vom Entwickler und/oder Kupplertyp in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels, enthält.

Geeignete Oxidationsfarbstoffvorprodukte vom Entwicklertyp sind beispielsweise p-Phenylendiamin und dessen Derivate. Bevorzugte p-Phenylendiamine werden ausgewählt aus einer oder mehrerer Verbindungen der Gruppe, welche gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, N,N-Bis-(2-hydroxyethyl)-p-phenylen-diamin, 2-(2-Hydroxyethyl)-p-phenylendiamin, 2-(1,2-Dihydroxyethyl)-p-phenylendiamin, N-(2-Hydroxypropyl)-p-phenylendiamin, N-(4'-Aminophenyl)-p-phenylendiamin, 2-Methoxymethyl-p-phenylendiamin, N-Phenyl-p-phenylendiamin, 2-(2-Hydroxyethyloxy)-p-phenylendiamin und N-(4-Amino-3-methyl-phenyl)-N-[3-(1H-imidazol-1-yl)propyl]amin sowie ihren physiologisch verträglichen Salzen.

Es kann erfindungsgemäß weiterhin bevorzugt sein, als Entwicklerkomponente Verbindungen einzusetzen, welche mindestens zwei aromatische Kerne enthalten, die mit Amino- und/oder Hydroxylgruppen substituiert sind. Bevorzugte zweikernige Entwicklerkomponenten werden ausgewählt aus N,N'-Bis-(2-hydroxyethyl)-N,N'-bis-(4'-aminophenyl)-1,3-diaminopropan-2-ol, N,N'-Bis-(4-aminophenyl)-1,4-diazacycloheptan, Bis-(2-hydroxy-5-aminophenyl)methan sowie deren physiologisch verträglichen Salzen.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-Aminophenol, 4-Amino-3-methylphenol, 4-Amino-2-aminomethyl-phenol, 4-Amino-2-(1,2-dihydroxyethyl)phenol, 4-Amino-2-(diethylaminomethyl)phenol sowie deren physiologisch verträglichen Salze.

Ferner kann die Entwicklerkomponente ausgewählt sein aus o-Aminophenol und dessen Derivaten, bevorzugt aus 2-Amino-4-methylphenol, 2-Amino-5-methylphenol, 2-Amino-4-chlorphenol und/oder deren physiologisch verträglichen Salzen.

Weiterhin kann die Entwicklerkomponente ausgewählt sein aus heterocyclischen Entwicklerkomponenten, wie Pyrimidin-Derivaten, Pyrazol-Derivaten, Pyrazolopyrimidin-Derivaten bzw. ihren physiologisch verträglichen Salzen. Bevorzugte Pyrimidin-Derivate sind 2,4,5,6-Tetraaminopyrimidin, 4-Hydroxy-2,5,6-triaminopyrimidin und deren physiologisch verträgliche Salze. Ein bevorzugtes Pyrazol-Derivat ist 4,5-Diamino-1-(2-hydroxyethyl)pyrazol sowie dessen physiologisch verträglichen Salze. Als Pyrazolopyrimidine sind insbesondere Pyrazolo[1,5-a]pyrimidine bevorzugt.

Bevorzugte Oxidationsfarbstoffvorprodukte vom Entwicklertyp sind ausgewählt aus der Gruppe, welche gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-amino-methylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin oder den physiologisch verträglichen Salzen dieser Verbindungen.

Besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)-propyl]amin, und/oder 4,5-Diamino-1-(2-hydroxyethyl)pyrazol sowie deren physiologisch verträglichen Salze.

Gemäß einer weiteren bevorzugten Ausführungsform der vorliegenden Erfindung enthält das erfindungsgemäße kosmetische Mittel als Oxidationsfarbstoffvorprodukt neben mindestens einer Entwicklerkomponente weiterhin zusätzlich mindestens eine Kupplerkomponente. Als Kupplerkomponenten werden in der Regel m-Phenylendiaminderivate, Naphthole, Resorcin und Resorcinderivate, Pyrazolone und m-Aminophenolderivate verwendet.

Erfindungsgemäß bevorzugte Kupplerkomponenten sind ausgewählt aus
(A) m-Aminophenol und dessen Derivaten, insbesondere 3-Aminophenol, 5-Amino-2-methylphenol, 3-Amino-2-chlor-6-methylphenol, 5-Amino-4-chlor-2-methylphenol, 5-(2'-Hydroxyethyl)-amino-2-methylphenol und 2,4-Dichlor-3-aminophenol,
(B) o-Aminophenol und dessen Derivaten, wie 2-Amino-5-ethylphenol,
(C) m-Diaminobenzol und dessen Derivaten, wie beispielsweise 2,4-Diaminophenoxy-ethanol, 1,3-Bis-(2',4'-diaminophenoxy)-propan, 1-Methoxy-2-amino-4-(2'-hydroxyethylamino)benzol, 2,6-Bis-(2'-hydroxyethylamino)-1-methylbenzol, 2-({3-[(2-Hydroxyethyl)amino]-4-methoxy-5-methylphenyl}amino)ethanol und 2-({3-[(2-Hydroxyethyl)amino]-2-methoxy-5-methylphenyl}-amino)ethanol,
(D) o-Diaminobenzol und dessen Derivaten,
(E) Di- beziehungsweise Trihydroxybenzolderivaten, insbesondere Resorcin, 2-Chlorresorcin, 4-Chlorresorcin, 2-Methylresorcin und 1,2,4-Trihydroxybenzol,
(F) Pyridinderivaten, insbesondere 3-Amino-2-methylamino-6-methoxypyridin, 2,6-Diaminopyridin, 2,6-Dihydroxy-3,4-dimethylpyridin, 2-Amino-3-hydroxypyridin und 3,5-Diamino-2,6-dimethoxypyridin,
(G) Naphthalinderivaten, wie 1-Naphthol und 2-Methyl-1-naphthol,
(H) Morpholinderivaten, wie 6-Hydroxybenzomorpholin,
(I) Chinoxalinderivaten,
(J) Pyrazolderivaten, wie 1-Phenyl-3-methylpyrazol-5-on,
(K) Indolderivaten, wie 6-Hydroxyindol,
(L) Pyrimidinderivaten oder
(M) Methylendioxybenzolderivaten, wie 1-(2'-Hydroxyethyl)-amino-3,4-methylendioxybenzol
sowie deren physiologisch verträglichen Salze.

Erfindungsgemäß bevorzugte Kupplerkomponenten sind ausgewählt aus der Gruppe, welche gebildet wird aus 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder den physiologisch verträglichen Salzen der vorgenannten Verbindungen.

Erfindungsgemäß besonders bevorzugte Kupplerkomponenten sind Resorcin, 2-Methylresorcin, 4-Chlorresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin, 2,6-Dihydroxy-3,4-dimethylpyridin, 1,5-Dihydroxynaphthalin, 2,7-Dihydroxynaphthalin und 1-Naphthol sowie deren physiologisch verträgliche Salze.

In einer besonders bevorzugten Ausführungsform der vorliegenden Erfindung sind die erfindungsgemäßen kosmetischen Mittel dadurch gekennzeichnet, dass sie als Oxidationsfarbstoffvorprodukt mindestens eine Entwicklerkomponente, ausgewählt aus der Gruppe von p-Phenylendiamin, p-Toluylendiamin, N,N-Bis-(2-hydroxyethyl)amino-p-phenylendiamin, 1,3-Bis-[(2-hydroxyethyl-4'-aminophenyl)amino]-propan-2-ol, 1,10-Bis-(2',5'-diaminophenyl)-1,4,7,10-tetraoxadecan, 4-Aminophenol, 4- Amino-3-methylphenol, Bis-(5- amino-2-hydroxyphenyl)methan, 2,4,5,6- Tetraaminopyrimidin, 2-Hydroxy-4,5,6-triaminopyrimidin, 4,5-Diamino-1-(2-hydroxyethyl)-pyrazol, deren physiologisch verträglichen Salze sowie deren Mischungen, und mindestens eine Kupplerkomponente, ausgewählt aus der Gruppe von Resorcin, 2-Methylresorcin, 5-Methyl-resorcin, 2,5-Dimethylresorcin, 4-Chlorresorcin, Resorcinmonomethylether, 5-Aminophenol, 5-Amino-2-methylphenol, 5-(2-Hydroxyethyl)amino-2-methylphenol, 3- Amino-4-chlor-2-methylphenol, 3-Amino-2-chlor-6-methylphenol, 3-Amino-2,4-Dichlorphenol, 2,4-Diaminophenoxyethanol, 2-Amino-4-(2'-hydroxyethyl)amino-anisolsulfat, 1,3-Bis-(2,4-diaminophenoxy)propan, 2-Amino-3-hydroxypyridin, 2-Methylamino-3-amino-6-methoxypyridin, 2,6-Dihydroxy-3,4-dimethylpyridin, 3,5-Diamino-2,6-dimethoxypyridin, 1-Naphthol, 2-Methyl-1-naphthol, 1,5-Dihydroxynaphthalin, 2,7-Dihydroxynaphthalin, 1-Phenyl-3-methylpyrazol-5-on, 2,6-Bis-[(2'-hydroxyethyl)amino]-toluol, 4-Hydroxyindol, 6-Hydroxyindol, 6-Hydroxybenzomorpholin, deren physiologisch verträglichen Salze sowie deren Mischungen, enthalten.

Um eine ausgewogene und subtile Nuancenausbildung zu erhalten, kann es im Rahmen der vorliegenden Erfindung auch vorgesehen sein, dass die erfindungsgemäßen kosmetischen Mittel zusätzlich mindestens einen direktziehenden Farbstoff enthalten. Bei direktziehenden Farbstoffen handelt sich um Farbstoffe, welche direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole.

Direktziehende Farbstoffe können in anionische, kationische und nichtionische direktziehende Farbstoffe unterteilt werden.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen. Bevorzugte kationische direktziehende Farbstoffe sind kationische Triphenylmethanfarbstoffe, wie Basic Blue 7, Basic Blue 26, Basic Violet 2 und Basic Violet 14 sowie aromatische Systeme, welche mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Bevorzugt enthält das erfindungsgemäße kosmetische Mittel die direktziehenden Farbstoffe in einer Gesamtmenge von 0,001 bis 10 Gew.-%, vorzugsweise von 0,01 bis 8 Gew.-%, bevorzugt von 0,1 bis 5 Gew.-%, insbesondere von 0,5 bis 3 Gew.-%, bezogen auf das Gesamtgewicht des kosmetischen Mittels. Als zweiten wesentlichen Bestandteil b) enthalten die erfindungsgemäßen kosmetischen Mittel mindestens ein spezielles Protein-Siloxan-Polymer. Das im Rahmen der vorliegenden Erfindung eingesetzte spezielle Protein-Siloxan-Polymer weist eine hinreichende Stabilität gegenüber verschiedenen pH-Werten sowie stark oxidierenden Substanzen auf und gewährleistet daher auch unter harschen chemischen Bedingungen, welche während einer oxidativen Haarfärbung auftreten, eine verbesserte Pflege der keratinischen Fasern. Aufgrund der hohen Stabilität des erfindungsgemäß eingesetzten Protein-Siloxan-Polymers ist eine hervorragende Lagerstabilität der erfindungsgemäßen kosmetischen Mittel gewährleistet.

Gemäß der vorliegenden Erfindung steht in der Struktureinheit der Formel (I) X für die Gruppe *-CH₂-CH(OH)-CH₂-O-(CH₂)₃-* steht, R¹ und R² jeweils für eine Hydroxylgruppe stehen und R³ für ein Wasserstoffatom.

Weiterhin wenn in der Struktureinheit der Formel (II) X für die Gruppe *-CH₂-CH(OH)-CH₂-O-(CH₂)₃-* steht, R¹ und R² jeweils für eine Hydroxylgruppe stehen und R³ für ein Wasserstoffatom steht.

Der Einsatz dieser speziellen Protein-Siloxan-Polymere resultiert in einer erhöhten Pflege der keratinischen Fasern, insbesondere einer erhöhten Nasskämmbarkeit, nach der Farbveränderung der keratinischen Fasern. Gleichzeitig weisen diese Protein-Siloxan-Polymere eine hinreichende Stabilität gegenüber den zur oxidativen Färbung verwendeten Oxidationsmitteln auf, so dass keine Zersetzung dieser Moleküle während der oxidativen Haarfärbung auftritt. Darüber hinaus weisen diese Protein-Siloxan-Polymere keine Inkompatibilitäten mit den üblicherweise in oxidativen Färbemitteln verwendeten Inhaltsstoffen auf, so dass die erfindungsgemäßen kosmetischen Mittel eine hervorragende Lagerstabilität besitzen.

Die erfindungsgemäß eingesetzten Protein-Siloxan-Polymere, insbesondere die Polymere gemäß den Strukturformeln (I), und (II), können durch Reaktion eines organofunktionellen Silikons, welches mindestens eine reaktive Gruppe, ausgewählt aus Acylhaliden, Sulphonylhaliden, Anhydriden, Aldehyden und Epoxgruppen, mit einem Proteinrest erhalten werden. Als reaktive Silikonkomponente können alle Verbindungen eingesetzt werden, welche eine Siloxangruppe (Si-O-Si) oder eine Silangruppe, welche durch Kondensation Siloxane bilden kann, enthalten. Erfindungsgemäß bevorzugt werden vernetzte Protein-Siloxan-Polymere eingesetzt. Die Vernetzung kann entweder durch Verwendung von polyfunktionellen Silikonverbindungen oder durch Verwendung von monofunktionellen Silikonverbindungen mit mindestens einer freien Silanolgruppe (Si-OH) erreicht werden.

Bevorzugt enthält das mindestens eine Protein-Siloxan-Polymer b) pro Aminogruppe des hydrolysierten Proteinrests R 0,1 bis 0,4, insbesondere 0,1 bis 0,3 Moleküle Siloxan. Besonders gute Pflegeeffekte werden erhalten, wenn das Protein-Siloxan-Polymer das zuvor beschriebene Verhältnis aufweist. Dieses Verhältnis kann durch Verwendung der entsprechenden Stöchiometrie des Proteinrestes zu der organofunktionellen Silikonverbindung erhalten werden. Hierfür werden zunächst die freien Aminogruppen des Proteins ermittelt, beispielsweise unter Verwendung von 2,4,6-Trinitrobenzolsulphonsäure als Bestimmungsreagenz (siehe Fields R., Biochem. J., 1971, 124, 581-590), und hieraus die erforderliche Menge der organofunktionellen Silikonverbindung zur Einstellung der obigen Verhältnisse bestimmt.

Weiterhin hat es sich als vorteilhaft erwiesen, wenn das mindestens eine Protein-Siloxan-Polymer b) 5 bis 30 %, vorzugsweise 5 bis 25 %, bevorzugt 10 bis 30 %, insbesondere 10 bis 15 %, mit Siloxanen umgesetzte Amingruppen und 70 bis 95 %, vorzugsweise 75 bis 95 %, bevorzugt 80 bis 95 %, insbesondere 85 bis 90 %, freie Aminogruppe enthält. Unter "freien Aminogruppen" sind Aminogruppen (NH₂-Gruppen) des Proteinrestes zu verstehen, welche nicht mit der organofunktionellen Silikonverbindung umgesetzt wurden und deshalb als freie Aminogruppe bzw. als protonierte Aminogruppe bei entsprechenden pH-Werten vorliegen. Die Bestimmung der freien Aminogruppen kann beispielsweise mittels der zuvor angeführten Methode erfolgen. Protein-Siloxan-Polymere, welche derartige Mengen an freien Aminogruppen aufweisen, haben sich als besonders wirkungsvoll im Hinblick auf die Verstärkung des Pflegeeffekts der erfindungsgemäßen kosmetischen Mittel erwiesen.

Als Proteinrest R kommt grundsätzlich jedes Protein in Frage, welches aus der Kartoffel isoliertes Proteinhydrolysat kommt.

Für den enzymatischen Abbau sind alle hydrolytisch wirkenden Enzyme geeignet, wie beispielsweise alkalische Proteasen. Übersichten zu Herstellung von Proteinhydrolysaten sind beispielsweise von G. Schuster und A. Domsch in Seifen Öle Fette Wachse 108, (1982) 177 bzw. Cosm.Toil. 99, (1984) 63, von H. W. Steisslinger in Parf.Kosm. 72, (1991) 556 und F. Aurich et al. in Tens.Surf.Det. 29, (1992) 389 erschienen. Mischungen von einzelnen Aminosäuren, welche lediglich durch Vermischen der Reinsubstanzen der Aminosäuren erhalten werden, sowie Totalhydrolysate, welche lediglich aus einzelnen Aminosäuren bestehen, fallen im Rahmen der vorliegenden Erfindung nicht unter den Begriff "hydrolysierte Proteine" bzw. "Proteinhydrolysate".

Besonders gute Ergebnisse im Rahmen der vorliegenden Erfindung werden jedoch erhalten, wenn Proteinhydrolysate eingesetzt werden, welche aus der Kartoffel isoliert werden können. Erfindungsgemäß einsetzbare Proteinhydrolysate aus der Kartoffel sind beispielsweise unter der Handelsbezeichnung Hydrosolanum der Firma Croda erhältlich und weisen mittlere Molekulargewichte M_{w} von etwa 750 Da auf.

Protein-Siloxan-Polymere, welche hydrolysierte Proteinreste mit den zuvor genannten mittleren Molekulargewichten M_{w} aufweisen, führen zu einer besonders guten Pflege der mit den erfindungsgemäßen kosmetischen Mitteln gefärbten keratinischen Fasern. Das mittlere Molekulargewicht M_{w} kann beispielsweise durch Gelpermeationschromatographie (GPC) bestimmt werden (Andrews P.; "Estimation of the Molecular Weights of Proteins by Sephadex Gel-Filtration"; Biochem. J., 1964, 91, Seiten 222 bis 233).

Weiterhin ist es im Rahmen der vorliegenden Erfindung vorteilhaft, wenn das mindestens eine Protein-Siloxan-Polymer b) ein mittleres Molekulargewicht M_{w} von 350 Da bis 90.000 Da, vorzugsweise von 600 bis 80.000 Da, bevorzugt von 1.000 bis 70.000 Da, insbesondere von 2.000 bis 64.000 Da, aufweist. Spezielle Protein-Siloxan-Polymere, welche das zuvor angeführte mittlere Molekulargewicht M_{w} aufweisen, resultieren in einer besonders hohen Pflege keratinischer Fasern nach der Farbveränderung. Das mittlere Molekulargewicht M_{w} kann beispielsweise durch Gelpermeationschromatographie (GPC) bestimmt werden (Liu X. M. et. al.; "Comparative Studies of Poly(DimethylSiloxanes) Using Automated GPC-MALDI-TOF MS and On-Line GPC-ESI-TOF MS"; Am. Soc. Mass. Spectrom., 2003, 14, Seiten 195 bis 202).

Das mindestens eine Protein-Siloxan-Polymer b) ist in den erfindungsgemäßen kosmetischen Mitteln in einer Gesamtmenge von 0,02 bis 0,2 Gew.-%, bezogen auf das Gesamtgewicht des kosmetischen Mittels, enthalten. Der Einsatz der zuvor genannten Gesamtmenge des speziellen Protein-Siloxan-Polymers führt zu einer erhöhten Pflege der keratinischen Fasern und stellt gleichzeitig eine hohe Lagerstabilität der erfindungsgemäßen kosmetischen Mittel sicher, da bei diesen Gesamtmengen keine unerwünschten Wechselwirkungen mit weiteren Inhaltsstoffen der kosmetischen Mittel auftreten.

Es hat sich gezeigt, dass ein Zusatz von unfunktionalisierten Proteinhydrolysaten im Rahmen der vorliegenden Erfindung besonders vorteilhaft ist. Erfindungsgemäß bevorzugte kosmetische Mittel enthalten daher zusätzlich mindestens ein unfunktionalisiertes Proteinhydrolysat, vorzugsweise ein aus der Gattung *Solanum* isoliertes unfunktionalisiertes Proteinhydrolysat, insbesondere ein aus Kartoffel isoliertes unfunktionalisiertes Proteinhydrolysat. Unter dem Begriff "unfunktionalisiertes Proteinhydrolysat" wird erfindungsgemäß ein Proteinhydrolysat verstanden, welches nach der Hydrolyse und gegebenenfalls Aufreinigung keiner weiteren Modifikation, insbesondere keiner chemischen und/oder physikalischen Modifikation, unterzogen wurde. Durch den Zusatz des mindestens einen unfunktionalisierten Proteinhydrolysats kann die Pflegewirkung der erfindungsgemäßen kosmetischen Mittel weitergehend gesteigert werden.

Die erfindungsgemäßen kosmetischen Mittel können weitere Wirk- und Zusatzstoffe enthalten. Es ist daher im Rahmen der vorliegenden Erfindung bevorzugt, wenn das kosmetische Mittel zusätzlich mindestens eine weitere Verbindung, ausgewählt aus der Gruppe von (i) Verdickungsmitteln; (ii) linearen oder verzweigten, gesättigten oder ungesättigten Alkoholen mit 8 bis 20 Kohlenstoffatomen; (iii) Tensiden, insbesondere amphoteren Tensiden; (iv) Alkalisierungsmitteln; (v) Ölen; sowie (vi) deren Mischungen, enthält.

Bevorzugt werden die erfindungsgemäßen kosmetischen Mittel als fließfähige Zubereitungen formuliert. Dabei sollten die erfindungsgemäßen kosmetischen Mittel so formuliert werden, dass diese sich einerseits gut am Anwendungsort auftragen und verteilen lassen, andererseits jedoch ausreichend viskos sind, so dass sie während der Einwirkzeit am Wirkort verbleiben und nicht verlaufen.

Es hat sich daher erfindungsgemäß als vorteilhaft erwiesen, wenn die erfindungsgemäßen kosmetischen Mittel mindestens ein Verdickungsmittel aus der Gruppe von (i) anionischen, synthetischen Polymeren; (ii) kationischen, synthetischen Polymeren; (iii) natürlich vorkommenden Verdickungsmitteln, wie nichtionischen Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektinen, Alginaten, Stärke-Fraktionen und Derivaten, wie Amylose, Amylopektin und Dextrinen, sowie Cellulosederivaten, wie beispielsweise Methylcellulose, Carboxyalkylcellulosen und Hydroxyalkylcellulosen; (iv) nichtionischen, synthetischen Polymeren, wie Polyvinylalkohol oder Polyvinylpyrrolidinon; (v) anorganischen Verdickungsmitteln, insbesondere Schichtsilikaten wie beispielsweise Bentonit, besonders Smektite, wie Montmorillonit oder Hectorit; sowie (vi) deren Mischungen, in einer Gesamtmenge von 0,0005 bis 5,0 Gew.-%, vorzugsweise von 0,001 bis 3,0 Gew.-%, bevorzugt von 0,005 bis 1,0 Gew.-%, insbesondere von 0,008 bis 0,01 Gew.-%, bezogen auf das Gesamtgewicht des kosmetischen Mittels, enthalten.

Es hat sich im Rahmen der vorliegenden Erfindung als besonders vorteilhaft erwiesen, wenn als Verdickungsmittel mindestens ein natürlich vorkommendes Verdickungsmittel, insbesondere Xanthangum sowie dessen Salze, in einer Gesamtmenge von 0,0005 bis 5,0 Gew.-%, vorzugsweise von 0,001 bis 1,0 Gew.-%, bevorzugt von 0,005 bis 0,5 Gew.-%, insbesondere von 0,01 bis 0,1 Gew.-%, bezogen auf das Gesamtgewicht des kosmetischen Mittels, enthalten ist.

Im Rahmen der vorliegenden Erfindung kann es bevorzugt sein, wenn der lineare oder verzweigte, gesättigte oder ungesättigte Alkohol mit 8 bis 20 Kohlenstoffatomen ausgewählt ist aus der Gruppe von Myristylalkohol (1-Tetradecanol), Stearylalkohol (1-Octadecanol), Cetearylalkohol, 2-Octyldodecanol, Arachylalkohol (Eicosan-1-ol), Gadoleylalkohol ((9Z)-Eicos-9-en-1-ol), Arachidonalkohol ((5Z,8Z,11Z,14Z)-Eicosa-5,8,11,14-tetraen-1-ol), vorzugsweise 2-Octyldodecanol und/oder Cetearylalkohol, und in einer Gesamtmenge von 1,0 bis 35 Gew.-%, vorzugsweise von 5,0 bis 30 Gew.-%, bevorzugt von 10 bis 25 Gew.-%, insbesondere von 12 bis 20 Gew.-%, bezogen auf das Gesamtgewicht des kosmetischen Mittels, enthalten ist.

Bevorzugt können die erfindungsgemäßen kosmetischen Mittel weiterhin mindestens einen Partialester aus einem Polyol mit 2 bis 6 Kohlenstoffatomen und linearen gesättigten Carbonsäuren mit 12 bis 30, insbesondere 14 bis 22 Kohlenstoffatomen, wobei die Partialester hydroxyliert sein können, in einer Gesamtmenge von 0,5 bis 10 Gew.-%, insbesondere von 3,0 bis 8,0 Gew.-%, bezogen auf das Gesamtgewicht des kosmetischen Mittels, enthalten. Solche Partialester sind insbesondere die Mono- und Diester von Glycerin oder die Monoester von Propylenglycol oder die Mono- und Diester von Ethylenglycol oder die Mono-, Di-, Tri- und Tetraester von Pentaerythrit jeweils mit linearen gesättigten C₁₂ - C₃₀-Carbonsäuren, welche hydroxyliert sein können, insbesondere diejenigen mit Palmitin- und Stearinsäure, die Sorbitanmono-, -di- oder -triester von linearen gesättigten C₁₂ - C₃₀-Carbonsäuren, welche hydroxyliert sein können, insbesondere diejenigen von Myristinsäure, Palmitinsäure, Stearinsäure oder von Mischungen dieser Fettsäuren und die Methylglucosemono- und -diester von linearen gesättigten C₁₂ - C₃₀-Carbonsäuren, welche hydroxyliert sein können.

Im Rahmen der vorliegenden Erfindung kann es vorgesehen sein, dass die erfindungsgemäßen kosmetischen Mittel mindestens einen Polyolpartialester, ausgewählt aus Glycerinmonostearat, Glycerinmonopalmitat, Glycerindistearat, Glycerindipalmitat, Ethylenglycolmonostearat, Ethylenglycolmonopalmitat, Ethylenglycoldistearat, Ethylenglycoldipalmitat, sowie Mischungen hiervon, insbesondere Mischungen aus Glycerinmonostearat, Glycerinmonopalmitat, Glycerindistearat und Glycerindipalmitat in einer Gesamtmenge von 0,5 bis 10 Gew.-%, insbesondere von 3,0 bis 8,0 Gew.-%, bezogen auf das Gesamtgewicht des kosmetischen Mittels, enthalten.

Der Einsatz der zuvor angeführten Alkohole, Partialester und Polypartialester in den erfindungsgemäßen kosmetischen Mitteln kann insbesondere dann bevorzugt sein, wenn die erfindungsgemäßen kosmetischen Mittel in Form einer Öl-in-Wasser-Emulsion vorliegen.

Weiterhin kann es erfindungsgemäß vorgesehen sein, dass die kosmetischen Mittel gemäß der Erfindung mindestens ein Tensid enthalten. Tenside im Sinne der vorliegenden Erfindung sind amphiphile (bifunktionelle) Verbindungen, welche aus mindestens einem hydrophoben und mindestens einem hydrophilen Molekülteil bestehen. Eine Basiseigenschaft von Tensiden und Emulgatoren ist die orientierte Absorption an Grenzflächen sowie die Aggregation zu Mizellen und die Ausbildung von lyotrophen Phasen.

Gemäß einer bevorzugten Ausführungsform der vorliegenden Erfindung enthalten die erfindungsgemäßen kosmetischen Mittel mindestens ein amphoteres Tensid in einer Gesamtmenge von 0,1 bis 5,0 Gew.-%, insbesondere von 0,2 bis 2,0 Gew.-%, bezogen auf das Gesamtgewicht des kosmetischen Mittels. Als amphotere bzw. zwitterionische Tenside werden solche oberflächenaktiven Verbindungen bezeichnet, welche mindestens eine quartäre Ammoniumgruppe und mindestens eine - COO⁽⁻⁾- oder -SO3⁽⁻⁾-Gruppe aufweisen.

Als amphotere Tenside sind im Rahmen der vorliegenden Erfindung die nachfolgend genannten Verbindungen besonders bevorzugt:
- Alkylbetaine mit 8 bis 20 Kohlenstoffatomen in der Alkylgruppe,
- Amidopropylbetaine mit 8 bis 20 Kohlenstoffatomen in der Acylgruppe,
- Sulfobetaine mit 8 bis 20 Kohlenstoffatomen in der Acylgruppe, und
- Amphoacetate oder Amphodiacetate mit 8 bis 20 Kohlenstoffatomen in der Acylgruppe.

In einer besonders bevorzugten Ausführungsform enthalten die erfindungsgemäßen kosmetischen Mittel als Tensid mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, bezogen auf das Gesamtgewicht des kosmetischen Mittels.

Weiterhin kann es vorgesehen sein, dass die erfindungsgemäßen kosmetischen Mittel mindestens ein ethoxyliertes nichtionisches Tensid in einer Gesamtmenge von 0,5 bis 6,0 Gew.-%, insbesondere von 1,0 bis 4,0 Gew.-%, bezogen auf das Gesamtgewicht des kosmetischen Mittels, enthalten. Dabei hat es sich als besonders vorteilhaft erwiesen, wenn das ethoxylierte nichtionische Tensid einen HLB-Wert oberhalb von 10, vorzugsweise oberhalb von 13 aufweist. Hierzu ist es erforderlich, dass das nichtionische Tensid einen ausreichend hohen Ethoxylierungsgrad besitzt. In diesem Zusammenhang enthält das erfindungsgemäße kosmetische Mittel daher als ethoxyliertes nichtionisches Tensid mindestens ein ethoxyliertes Tensid mit mindestens 12 Ethylenoxideinheiten. Neben den entsprechend ethoxylierten Fettalkoholen, insbesondere Laurylalkohol, Myristylalkohol, Cetylalkohol, Stearylalkohol, Arachylalkohol und Behenylalkohol, sind erfindungsgemäß insbesondere die Anlagerungsprodukte von 20 bis 60 Mol Ethylenoxid an Rizinusöl und gehärtetes Rizinusöl besonders geeignet. Das mindestens eine ethoxylierte nichtionische Tensid ist bevorzugt ausgewählt aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30.

Kosmetische Mittel im Rahmen der vorliegenden Erfindung weisen in der Regel einen basischen pH-Wert, insbesondere zwischen pH 8,0 und pH 12, auf. Diese pH-Werte sind erforderlich, um eine Öffnung der äußeren Schuppenschicht (Cuticula) zu gewährleisten und eine Penetration der Oxidationsfarbstoffvorprodukte und/oder des Oxidationsmittels in das Haar zu ermöglichen.

Die Einstellung des zuvor genannten pH-Wertes kann bevorzugt unter Verwendung eines Alkalisierungsmittels erfolgen. Im Rahmen der vorliegenden Erfindung ist das Alkalisierungsmittel ausgewählt aus der Gruppe von (i) anorganischen Alkalisierungsmitteln; (ii) organischen Alkalisierungsmitteln; sowie (iii) deren Mischungen, und in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels, enthalten.

Bevorzugte anorganische Alkalisierungsmittel sind ausgewählt aus der Gruppe, welche gebildet wird aus Ammoniak bzw. Ammoniumhydroxid, also wässrigen Lösungen von Ammoniak, Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat sowie Mischungen hiervon. Ammoniak bzw. Ammoniumhydroxid ist ein besonders bevorzugtes Alkalisierungsmittel. Besonders bevorzugt ist Ammoniak in einer Gesamtmenge von 0,1 bis 20 Gew.-%, vorzugsweise von 0,5 bis 10 Gew.-%, insbesondere von 1,0 bis 7,0 Gew.-%, bezogen auf das Gesamtgewicht des kosmetischen Mittels, enthalten.

Bevorzugte organische Alkalisierungsmittel sind ausgewählt aus mindestens einem Alkanolamin. Erfindungsgemäß bevorzugte Alkanolamine sind ausgewählt aus Alkanolaminen aus primären, sekundären oder tertiären Aminen mit einem C₂-C₆-Alkylgrundkörper, welcher mindestens eine Hydroxylgruppe trägt. Besonders bevorzugte Alkanolamine werden aus der Gruppe ausgewählt, welche gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol (Monoisopropanolamin), 1-Aminobutan-2-ol, 1-Aminopentan-2-ol, 1-Aminopentan-3-ol, 1-Aminopentan-4-ol, 2-Amino-2-methyl-propanol, 2-Amino-2-methylbutanol, 3-Amino-2-methylpropan-1-ol, 1-Amino-2-methylpropan-2-ol, 3-Aminopropan-1,2-diol, 2-Amino-2-methylpropan-1,3-diol, 2-Amino-2-ethyl-1,3-propandiol, N,N-Dimethyl-ethanolamin, Triethanolamin, Diethanolamin und Triisopropanolamin. Erfindungsgemäß ganz besonders bevorzugte Alkanolamine werden ausgewählt aus der Gruppe von 2-Aminoethan-1-ol (Monoethanolamin), 2-Amino-2-methyl-propan-1-ol, 2-Amino-2-methyl-propan-1,3-diol und Triethanolamin. Besonders bevorzugte erfindungsgemäße kosmetische Mittel enthalten eine Mischung aus Monoethanolamin und 2-Amino-2-methylpropan-1ol. Bevorzugt ist das mindestens eine Alkanolamin in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels, enthalten.

Weitere erfindungsgemäß bevorzugte organische Alkalisierungsmittel sind ausgewählt aus basischen Aminosäuren, besonders bevorzugt ausgewählt aus der Gruppe, welche gebildet wird aus L-Arginin, D-Arginin, D/L-Arginin, L-Lysin, D-Lysin, D/L-Lysin sowie Mischungen hiervon. Erfindungsgemäß besonders bevorzugte basische Aminosäuren sind ausgewählt aus L-Arginin, D-Arginin und D/L-Arginin. Bevorzugte erfindungsgemäße kosmetische Mittel enthalten mindestens ein von Alkanolaminen und Ammoniak verschiedenes Alkalisierungsmittel in einer Gesamtmenge von 0,05 bis 5,0 Gew.-%, insbesondere von 0,5 bis 3,0 Gew.-%, bezogen auf das Gesamtgewicht des kosmetischen Mittels.

Erfindungsgemäß bevorzugt ist das Alkalisierungsmittel ausgewählt aus der Gruppe von Natriumhydroxid, Kaliumhydroxid, Ammoniak, Monoethanolamin und 2-Amino-2-methylpropan, vorzugsweise Monoethanolamin, und ist in einer Gesamtmenge von 1,5 bis 9,5 Gew.-%, vorzugsweise von 2,5 bis 8,5 Gew.-%, bevorzugt von 3,0 bis 8,0 Gew.-%, insbesondere von 3,5 bis 7,5 Gew.-%, bezogen auf das Gesamtgewicht des kosmetischen Mittels, enthalten.

In einer besonders bevorzugten Ausführungsform enthalten die erfindungsgemäßen kosmetischen Mittel als Alkalisierungsmittel eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, bezogen auf das Gesamtgewicht des kosmetischen Mittels.

Bevorzugt beträgt der pH-Wert der erfindungsgemäßen kosmetischen Mittel, gemessen bei 22°C, 8 bis 13, vorzugsweise 9,5 bis 12, bevorzugt 10 bis 11,5, insbesondere 10,5 bis 11.

Im Rahmen der vorliegenden Erfindung kann es weiterhin bevorzugt sein, wenn die erfindungsgemäßen kosmetischen Mittel mindestens ein Öl, ausgewählt aus der Gruppe von Sonnenblumenöl, Maisöl, Sojaöl, Kürbiskernöl, Traubenkernöl, Sesamöl, Haselnussöl, Aprikosenkernöl, Macadamianussöl, Araraöl, Rizinusöl, Avocadoöl sowie deren Mischungen, in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels, enthalten. Durch den Einsatz mindestens eines zuvor genannten Öls kann der Pflegeeffekt der aminierten Siliconpolymere weitergehend gesteigert werden. Besonders bevorzugt enthalten die erfindungsgemäßen kosmetischen Mittel Traubenkernöl in einer Gesamtmenge von 0,1 bis 10 Gew.-%, bevorzugt von 0,2 bis 5,0 Gew.-%, insbesondere von 0,5 bis 2,0 Gew.-%, bezogen auf das Gesamtgewicht des kosmetischen Mittels.

Gemäß einer besonders bevorzugten Ausführungsform der vorliegenden Erfindung enthalten die als Öl-in-Wasser-Emulsion vorliegenden erfindungsgemäßen kosmetischen Mittel - bezogen auf das Gesamtgewicht der kosmetischen Mittel -
- Octyldodecanol in einer Gesamtmenge von 2,0 bis 20 Gew.-%, insbesondere von 5,0 bis 12 Gew.-%, weiterhin
- Mischungen aus Glycerinmonostearat, Glycerinmonopalmitat, Glycerindistearat und Glycerindipalmitat in einer Gesamtmenge von 0,5 bis 10 Gew.-%, bevorzugt 3,0 bis 8,0 Gew.-%, weiterhin
- mindestens ein amphoteres Tensid, ausgewählt aus Amidopropylbetainen mit 9 bis 13 Kohlenstoffatomen in der Acylgruppe, in einer Gesamtmenge von 0,1 bis 5,0 Gew.-%, insbesondere von 0,2 bis 2,0 Gew.-%, weiterhin
- eine Mischung aus mindestens zwei voneinander verschiedenen Alkanolaminen, insbesondere von Monoethanolamin und 2-Amino-2-methylpropan-1-ol, in einer Gesamtmenge von 0,05 bis 15 Gew.-%, vorzugsweise von 0,5 bis 10 Gew.-%, insbesondere von 3,5 bis 7,5 Gew.-%, weiterhin
- Traubenkernöl in einer Gesamtmenge von 0,1 bis 10 Gew.-%, bevorzugt von 0,2 bis 5,0 Gew.-%, insbesondere von 0,5 bis 2,0 Gew.-%.

Das erfindungsgemäß bevorzugt eingesetzte mindestens eine Protein-Siloxan-Polymer kann beispielsweise durch Umsetzung eines hydrolysierten Proteinrest R mit 1-[3-(2-Oxiranylmethoxy)propyl] -silantriol oder 2-[[3-(Trimethoxysilyl)propoxy]methyl]-oxiran erhalten werden. Für die Herstellung von vernetzten Protein-Siloxan-Polymeren ist im Falle des 2-[[3-(Trimethoxysilyl)propoxy]methyl]-oxiran zunächst eine Hydrolyse der Methoxygruppen erforderlich, wohingegen bei Verwendung des 1-[3-(2-Oxiranylmethoxy)propyl]-silantriol die Vernetzung ohne weitere Hydrolysereaktionen möglich ist. Ein im Rahmen der vorliegenden Erfindung einsetzbares Oxiran ist beispielsweise unter der Handelsbezeichnung Silquest A187 von der Firma Momentive Inc. kommerziell erhältlich.

Oxidative Färbemittel können auch direkt vor der Anwendung aus zwei oder mehreren getrennt verpackten Zusammensetzungen hergestellt werden. Dies bietet sich insbesondere zur Trennung inkompatibler Inhaltsstoffe an, um eine vorzeitige Reaktion zu vermeiden. Eine Auftrennung in Mehrkomponentensysteme ist insbesondere dort bevorzugt, wo Inkompatibilitäten der Inhaltsstoffe zu erwarten oder zu befürchten sind. Das oxidative Färbemittel wird in diesen Fällen vom Verbraucher direkt vor der Anwendung durch Vermischen der Komponenten hergestellt. Im Rahmen der vorliegenden Erfindung ist dieses Vorgehen bei oxidativen Färbemitteln, bei welchen das erfindungsgemäße kosmetische Mittel zunächst getrennt von einer Oxidationsmittelzubereitung, enthaltend mindestens ein Oxidationsmittel, vorliegt, besonders bevorzugt.

Unter dem Begriff "Container" wird im Rahmen der vorliegenden Erfindung eine Umhüllung verstanden, welche in Form einer gegebenenfalls wieder verschließbaren Flasche, einer Tube, einer Dose, eines Tütchens, eines Sachets oder ähnlichen Umhüllungen vorliegt. Dem Umhüllungsmaterial sind erfindungsgemäß keine Grenzen gesetzt. Bevorzugt handelt es sich dabei jedoch um Umhüllungen aus Glas oder Kunststoff.

Die Oxidationsmittel im Rahmen der vorliegenden Erfindung sind von Luftsauerstoff verschieden. Als Oxidationsmittel können Wasserstoffperoxid sowie dessen feste Anlagerungsprodukte an organische und anorganische Verbindungen eingesetzt werden. Als feste Anlagerungsprodukte kommen erfindungsgemäß insbesondere die Anlagerungsprodukte an Harnstoff, Melamin, Polyvinylpyrrolidinon sowie Natriumborat in Frage. Wasserstoffperoxid und/oder eines seiner festen Anlagerungsprodukte an organische oder anorganische Verbindungen sind als Oxidationsmittel besonders bevorzugt. Erfindungsgemäß bevorzugt ist das Oxidationsmittel daher ausgewählt aus der Gruppe von Persulfaten, Chloriten, Wasserstoffperoxid und Anlagerungsprodukten von Wasserstoffperoxid an Harnstoff, Melamin sowie Natriumborat, insbesondere Wasserstoffperoxid.

Ein besonders bevorzugte Ausführungsform der vorliegenden Erfindung ist somit dadurch gekennzeichnet, dass als Oxidationsmittel Wasserstoffperoxid in einer Gesamtmenge von 0,5 bis 7,0 Gew.-%, vorzugsweise von 1,0 bis 7,0 Gew.-%, insbesondere von 3,0 bis 7,0 Gew.-%, bezogen auf das Gesamtgewicht der Oxidationsmittelzubereitung, enthalten ist. Die Berechnung der Gesamtmenge bezieht sich hierbei auf 100 %-iges H₂O₂.

Die Oxidationsmittelzubereitungen können weiterhin Wasser in einer Gesamtmenge von 40 bis 98 Gew.-%, insbesondere von 65 bis 85 Gew.-%, bezogen auf das Gesamtgewicht der Oxidationsmittelzubereitung, enthalten.

Gemäß einer bevorzugten Ausführungsform der vorliegenden Erfindung enthalten die Oxidationsmittelzubereitungen weiterhin mindestens ein lineares gesättigtes Alkanol mit 12 bis 30 Kohlenstoffatomen, insbesondere mit 16 bis 22 Kohlenstoffatomen, in einer Gesamtmenge von 0,1 bis 10 Gew.-%, vorzugsweise von 0,5 bis 5,0 Gew.-%, insbesondere von 1,0 bis 4,0 Gew.-%, bezogen auf das Gesamtgewicht der Oxidationsmittelzubereitung. Bevorzugt sind insbesondere Cetylalkohol, Stearylalkohol, Arachidylalkohol, Behenylalkohol und Lanolinalkohol oder Gemische dieser Alkohole, wie sie bei der großtechnischen Hydrierung von pflanzlichen und tierischen Fettsäuren erhältlich sind, sowie Mischungen dieser Alkanole. Besonders bevorzugt ist die Mischung Cetearylalkohol.

In einer weiteren bevorzugten Ausführungsform der vorliegenden Erfindung enthalten die Oxidationsmittelzubereitungen mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1 bis 4,0 Gew.-%, bezogen auf das Gesamtgewicht der Oxidationsmittelzubereitung.

Im Rahmen der vorliegenden Erfindung kann es darüber hinaus auch vorgesehen sein, dass die Oxidationsmittelzubereitungen mindestens einen Ester aus einer Carbonsäure mit 10 bis 20 Kohlenstoffatomen und einem linearen oder verzweigten Alkohol mit 1 bis 5 Kohlenstoffatomen, insbesondere Isopropylmyristat, in einer Gesamtmenge von 3,0 bis 25 Gew.-%, vorzugsweise von 5,0 bis 20 Gew.-%, insbesondere von 8,0 bis 15 Gew.-%, bezogen auf das Gesamtgewicht der Oxidationsmittelzubereitung, enthalten.

Gemäß einer besonders bevorzugten Ausführungsform der vorliegenden Erfindung enthalten die Oxidationsmittelzubereitungen - bezogen auf das Gesamtgewicht der Oxidationsmittelzubereitungen -
- mindestens ein lineares gesättigtes Alkanol mit 12 bis 30 Kohlenstoffatomen in einer Gesamtmenge von 0,1 bis 10 Gew.-%, vorzugsweise von 0,5 bis 5,0 Gew.-%, insbesondere von 1,0 bis 4,0 Gew.-%, weiterhin
- mindestens ein ethoxyliertes nichtionisches Tensid, welches bevorzugt ausgewählt ist aus Tensiden mit der INCI-Bezeichnung Ceteth-12, Steareth-12, Ceteareth-12, Ceteth-20, Steareth-20, Ceteareth-20, Ceteth-30, Steareth-30, Ceteareth-30, Oleth-30, Ceteareth-50, PEG-40 Hydrogenated Castor Oil und PEG-60 Hydrogenated Castor Oil sowie Mischungen dieser Substanzen, besonders bevorzugt ausgewählt aus Ceteth-20, Steareth-20, Ceteareth-20, Ceteth-30, Steareth-30 und Ceteareth-30, in einer Gesamtmenge von 0,1 bis 10 Gew.-%, vorzugsweise von 0,5 bis 5,0 Gew.-%, insbesondere von 1,0 bis 4,0 Gew.-%, sowie
- mindestens einen Ester aus einer Carbonsäure mit 10 bis 20 Kohlenstoffatomen und einem linearen oder verzweigten Alkohol mit 1 bis 5 Kohlenstoffatomen, bevorzugt Isopropylmyristat, in einer Gesamtmenge von 3,0 bis 25 Gew.-%, vorzugsweise von 5,0 bis 20 Gew.-%, insbesondere von 8,0 bis 15 Gew.-%.

Die erfindungsgemäßen Oxidationsmittelzubereitungen enthalten weiterhin mindestens eine Säure. Bevorzugte Säuren sind ausgewählt aus Dipicolinsäure, Genusssäuren, wie beispielsweise Zitronensäure, Essigsäure, Apfelsäure, Milchsäure und Weinsäure, verdünnten Mineralsäuren, wie Salzsäure, Phosphorsäure, Pyrophosphorsäure und Schwefelsäure, sowie Mischungen hiervon. Die Oxidationsmittelzubereitungen weisen bevorzugt einen pH-Wert im Bereich von 2 bis 5, insbesondere von 3 bis 4, auf.

Zur Herstellung von oxidativen Färbemitteln aus der erfindungsgemäßen Verpackungseinheit (Kit-of-parts) wird das erfindungsgemäße kosmetische Mittel in dem Container C1 mit der Oxidationsmittelzubereitung in dem Container C2 oder *vice versa* vermischt.

Weiterhin kann es erfindungsgemäß besonders vorteilhaft sein, wenn die Verpackungseinheit mindestens ein weiteres Haarbehandlungsmittel, insbesondere eine Konditioniermittelzubereitung, in einem zusätzlichen Container enthält. Diese Konditioniermittelzubereitung enthält vorteilhafterweise mindestens ein Konditioniermittel, ausgewählt aus der Gruppe von kationischen Polymeren, Siliconderivaten und Ölen. Darüber hinaus kann die Verpackungseinheit Applikationshilfen, wie Kämme, Bürsten, Applicetten oder Pinsel, persönliche Schutzkleidung, insbesondere Ein-Weg-Handschuhe, sowie gegebenenfalls eine Gebrauchsanleitung umfassen. Unter einer Applicette wird ein breiter Pinsel verstanden, an dessen Stielende sich eine Spitze befindet, welche das Abteilen von Faserbündeln bzw. Strähnchen aus der Gesamtmenge der Fasern erlaubt und vereinfacht.

Bezüglich des erfindungsgemäßen kosmetischen Mittels in dem Container C1 und der Oxidationsmittelzubereitung in dem Container C2 gilt mutatis mutandis das zu den erfindungsgemäßen kosmetischen Mitteln Gesagte.

Ein Verfahren zur Färbung keratinischer Fasern, umfasst:
a) Bereitstellen eines erfindungsgemäßen kosmetischen Mittels (M1),
b) Bereitstellen einer Oxidationsmittelzubereitung (M2), enthaltend in einem kosmetisch verträglichen Träger mindestens ein Oxidationsmittel in einer Gesamtmenge von 0,5 bis 7,0 Gew.-%, vorzugsweise von 1,0 bis 7,0 Gew.-%, insbesondere von 3,0 bis 7,0 Gew.-%, insbesondere von bezogen auf das Gesamtgewicht der Oxidationsmittelzubereitung, sowie mindestens eine Säure,
c) Vermischen des kosmetischen Mittels (M1) mit der Oxidationsmittelzubereitung (M2),
d) Applizieren der in Schritt c) erhaltenen Mischung auf die keratinischen Fasern und Belassen dieser Mischung für eine Zeit von 10 bis 60 Minuten, bevorzugt von 20 bis 45 Minuten, bei Raumtemperatur und/oder bei mindestens 30 °C auf den keratinischen Fasern,
e) Spülen der keratinischen Fasern mit Wasser und/oder einer reinigenden Zusammensetzung für 1 bis 5 Minuten, und
f) gegebenenfalls Applizieren eines Nachbehandlungsmittels auf die keratinischen Fasern und Ausspülen nach einer Zeit von 1 bis 10 Minuten.

Das Verfahren zur Färbung keratinischer Fasern unter Verwendung eines speziellen Protein-Siloxan-Polymers resultiert in einer verbesserten Pflege gefärbter keratinischer Fasern.

Unter Raumtemperatur ist dabei im Rahmen der vorliegenden Erfindung die Umgebungstemperatur zu verstehen, welche ohne Einwirkung von externer Wärme vorherrscht und bevorzugt von 10 bis 39 °C beträgt. Die Wirkung der Färbezusammensetzung kann durch externe Wärmezufuhr, beispielsweise mittels einer Wärmehaube, verstärkt werden. Die bevorzugte Einwirkdauer der Färbezusammensetzung auf die keratinischen Fasern beträgt 10 bis 60 min, bevorzugt 20 bis 45 min. Nach Beendigung der Einwirkdauer wird das verbliebene Färbemittel aus den keratinischen Fasern mit Hilfe einer Reinigungszubereitung, welche bevorzugt mindestens ein kationisches und/oder anionisches und/oder nichtionisches Tensid enthält, und/oder Wasser ausgewaschen. Gegebenenfalls wird der Vorgang mit einem weiteren Mittel wiederholt. Nach dem Auswaschen werden die keratinischen Fasern gegebenenfalls mit einem Nachbehandlungsmittel, beispielsweise einem Konditioniermittel, gespült und mit einem Handtuch oder einem Heißluftgebläse getrocknet. Die Auftragung der Färbezusammensetzung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex zur einmaligen Benutzung (Einweghandschuhe) sowie gegebenenfalls eine Schürze. Es ist aber auch möglich, die Färbemittel mit einer Applikationshilfe auf die keratinischen Fasern aufzutragen.

Besonders bevorzugte erfindungsgemäße Verfahren sind dadurch gekennzeichnet, dass die Verfahren in einer verbesserten Pflege der keratinischen Fasern resultieren. Durch die Verwendung mindestens eines speziellen Protein-Siloxan-Polymers ist die aus dem erfindungsgemäßen Verfahren resultierende Pflegewirkung größer als die Pflegewirkung, welche in Abwesenheit des erfindungsgemäß eingesetzten Protein-Siloxan-Polymers b) erreicht werden kann.

Bezüglich des erfindungsgemäßen kosmetischen Mittels M1, der Oxidationsmittelzubereitung M2 sowie weiterer bevorzugter Ausführungsformen des erfindungsgemäßen Verfahrens gilt mutatis mutandis das zu den erfindungsgemäßen kosmetischen Mitteln sowie zu der erfindungsgemäßen Verpackungseinheit Gesagte.

Zudem ist ein weiterer Gegenstand der vorliegenden Erfindung die Verwendung eines erfindungsgemäßen kosmetischen Mittels zur Erhöhung der Pflege keratinischer Fasern. Der Einsatz eines speziellen Protein-Siloxan-Polymers resultiert in einer erhöhten Pflege gefärbter keratinischer Fasern.

Bezüglich weiterer bevorzugter Ausführungsformen der erfindungsgemäßen Verwendung gilt mutatis mutandis das zu den erfindungsgemäßen kosmetischen Mitteln sowie zu der erfindungsgemäßen Verpackungseinheit Gesagte.

Zudem ist ein weiterer Gegenstand der vorliegenden Erfindung die Verwendung einer erfindungsgemäßen Verpackungseinheit zur Herstellung eines kosmetischen Mittels zur Farbveränderung keratinischer Fasern mit erhöhter Pflege der keratinischen Fasern. Der Einsatz eines speziellen Protein-Siloxan-Polymers resultiert in einer erhöhten Pflege gefärbter keratinischer Fasern.

Bezüglich weiterer bevorzugter Ausführungsformen der erfindungsgemäßen Verwendung gilt mutatis mutandis das zu den erfindungsgemäßen kosmetischen Mitteln sowie zu der erfindungsgemäßen Verpackungseinheit Gesagte.

Die nachfolgenden Beispiele sollen bevorzugte Ausführungsformen der Erfindung erläutern, ohne sie jedoch einzuschränken.

### Beispiele:

### 1. Rezepturen

Zusammensetzungen der eingesetzten kosmetischen Mittel (Öl-in-Wasser-Emulsionen, alle Mengen in Gew.-%). Das in den nachfolgenden Formulierungen verwendete Protein-Siloxan-Polymer ist bevorzugt ein Protein-Siloxan-Polymer der Formeln (IV) und/oder (V) mit R¹ und R², jeweils unabhängig voneinander, Methyl und/oder einem Rest (VIa) oder (VIb) und einem mittleren Molekulargewicht M_{w} von 2.000 bis 64.000 Da.

| Rohstoff | V1 | E1* | E2* |
|---|---|---|---|
| Xanthan Gum | 0,05 | 0,05 | 0,05 |
| 2-Octyldodecanol | 2,3 | 2,3 | 2,3 |
| Lanette N ^{a)} | 14 | 14 | 14 |
| Cetearyl Alkohol | 3,9 | 3,9 | 3,9 |
| Glycerinmonostearat | 6,0 | 6,0 | 6,0 |
| Glycerol 99,5 % | 2,0 | 2,0 | 2,0 |
| Kokosamidopropylbeatin, 40%ig | 2,0 | 2,0 | 2,0 |
| Monoethanolamin | 4,5 | 4,5 | 4,5 |
| 2-Amino-2-methylpropanol | 0,1 | 0,1 | 0,1 |
| Natriumsulfit, wasserfrei | 0,2 | 0,2 | 0,2 |
| Caramelsirup, 75%ig | 0,1 | 0,1 | 0,1 |
| Traubenkernöl | 1,0 | 1,0 | 1,0 |
| p-Toluylendiaminsulfat | 0,03 | 0,03 | 0,03 |
| 4-Amino-3-methylphenol | 0,3 | 0,3 | 0,3 |
| 1,3-Benzoldiol | 0,04 | 0,04 | 0,04 |
| 1-Naphthol | 0,09 | 0,09 | 0,09 |
| 5-Amino-2-methylphenol | 0,2 | 0,2 | 0,2 |
| 2-Amino-6-chloro-4-nitrophenol | 0,2 | 0,2 | 0,2 |
| Protein-Siloxan-Polymer ** | - | 0,21 | 0,42 |
| Wasser, vollentsalzt | ad 100,00 | ad 100,00 | ad 100,00 |

| | | | |
|---|---|---|---|
| * erfindungsgemäß ** Aktivsubstanz ^{a)} INCI-Bezeichnung: Cetearyl alcohol, Sodium cetearyl sulfate (BASF) | | | |

Die Fettbasis wurde jeweils zusammen bei 80°C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt. Bei der Rezeptur V1 handelt es sich um eine nicht erfindungsgemäße Vergleichsrezeptur ohne Protein-Silicon-Polymer. Die Rezepturen E1 und E2 sind erfindungsgemäße Beispiele.

Oxidationsmittelzubereitung 01 (alle Mengen in Gew.-%)

| Rohstoff | O1 |
|---|---|
| Dinatriumpyrophosphat | 0,10 |
| Dipicolinsäure | 0,10 |
| Kaliumhydroxid 50% | 0,22 |
| 1-Hydroxyethan-1,1-diphosphonsäure 60% | 0,25 |
| Emulgade F ^{b)} | 4,0 |
| Cetearyl Alcohol | 0,5 |
| Ceteareth-20 | 0,5 |
| Bienenwachs | 0,3 |
| Isopropylmyristat | 10 |
| Wasserstoffperoxid 50 % | 11,2 |
| Wasser vollentsalzt | ad 100 |

| | |
|---|---|
| ^{b)} INCI-Bezeichnung: Cetearyl alcohol, PEG-40 Castor oil, Sodium cetearyl sulfat (BASF) | |

### 2. Verbesserte Pflege durch Zusatz mindestens eines speziellen Protein-Siloxan-Polymers

Zur Herstellung der oxidativen Färbemittel zur Bestimmung der Pflege wurden die kosmetischen Mittel V1, E1 und E2 jeweils im Gewichtsverhältnis 1:1 mit der obigen Oxidationsmittelzubereitung 01 vermischt.

12 Strähnen von natürlich hellbraunem europäischen Haar (IHIP (New York), lot # 03/2012, N104, length 15 cm, weight 1 g) wurden mit einer wässrigen Natrium-Laurylethersulfat-Lösung (3 % Aktivsubstanzgehalt in der Lösung) gewaschen. Die Strähnen wurden an der Luft getrocknet und für 24 h bei 25°C und 25% relativer Luftfeuchtigkeit gelagert. Nach Einweichen dieser Strähnen für 5 Minuten in Wasser wurde deren Nasskämmbarkeit bestimmt (Referenzwert).

Für die Färbungen wurden jeweils 12 Strähnen von natürlich europäischen Haar (IHIP (New York), lot # 03/2012, N104, length 15 cm, weight 1 g) je oxidativem Färbemittel verwendet. Hierfür wurden jeweils 4 g der zuvor hergestellten oxidativen Färbemittel pro 1 g Haarsträhne appliziert. Nachdem die Strähnen für 30 min bei 32 °C gefärbt wurden, wurden sie für 2 min mit Wasser gespült und an der Luft getrocknet.

Die Messung der Nasskämmbarkeit wurde wie folgt durchgeführt:
Vor der Messung wurde jede Strähne unter Kämmen mit einem harten Gummikamm mit feinen Zähnen (Firma Hercules Sägemann, Hamburg Germany) für 2 Sekunden mit Wasser befeuchtet. Nachdem 3 Kämmvorgänge durchgeführt wurden, wird die Kämmkraft während weiteren 10 Kämmvorgängen gemessen, wobei die jeweilige Haarsträhne während des Kämmvorgangs langsam rotiert. Die erhaltenen Messwerte werden unter Verwendung der folgenden in der Software Statistica 10.0 (StatSoft Inc., USA) eingebetteten statistischen Tests verglichen:
- Shapiro-Wilks Test (Test für Normalverteilung)
- Ausreißertest nach Grubbs
- Bartlett-Test (Test auf Homoskedastizität von Varianzen)
- Univarianter Signifikanztest
- Newman-Keuls Test (Bestimmung von signifikanten Unterschieden)
- Unequal N HSD Test (Test auf Mehrfachvergleiche).

Die Änderung der Kämmkraft dK in Prozent kann mit Hilfe der Formel dK = [(K₀-Kᵢ)/K₀]*100 berechnet werden. Ko ist hierbei der Mittelwert der Kämmkraft für die ungefärbten Haarsträhnen und Kᵢ der Mittelwert für die mit dem jeweiligen oxidativen Färbemittel behandelten Haarsträhnen.

Die Pflege der Haarsträhnen ist umso höher, je geringer die aufgewendete Kämmkraft und damit je höher die Änderung der Kämmkraft ist. In nachfolgender Tabelle sind die dK-Werte für die Ausfärbungen unter Verwendung der kosmetischen Mittel V1, E1 und E2 dargestellt. Die Ausfärbungen mit den erfindungsgemäßen kosmetischen Mitteln E1 und E2, welche mindestens ein spezielles Protein-Siloxan-Polymer in einer Gesamtmenge von 0,21 Gew.-% bzw. 0,42 Gew.-% enthalten, weisen im Vergleich zu den Ausfärbungen ohne Protein-Siloxan-Polymer (V1) eine höhere Änderung der Kämmkraft und somit eine erhöhte Pflege auf.

| Oxidatives Färbemittel | dK [%] |
|---|---|
| V1 + O1 (1:1) | 21 |
| E1 + O1 (1:1) | 30 |
| E2 + O1 (1:1) | 26 |

## Patentansprüche

1. Verpackungseinheit (Kit-of-Parts), umfassend - getrennt voneinander konfektioniert -
a) mindestens einen Container (C1), enthaltend ein kosmetisches Mittel zur Farbveränderung keratinischer Fasern, umfassend in einem kosmetisch verträglichen Träger
- mindestens eine Verbindung, ausgewählt aus der Gruppe von Oxidationsfarbstoffvorprodukten,
- mindestens ein Protein-Siloxan-Polymer in einer Gesamtmenge von 0,02 bis 0,2 Gew.-%, bezogen auf das Gesamtgewicht des kosmetischen Mittels, enthaltend mindestens eine Struktureinheit der Formel (I) und/oder mindestens eine Struktureinheit der Formel (II) 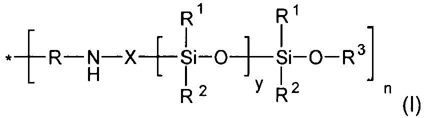 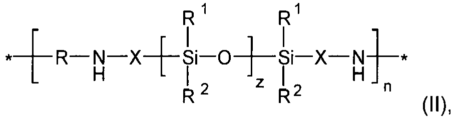 worin
R für ein aus der Kartoffel isoliertes Proteinhydrolysat mit mindestens einer NH₂-Gruppe steht,
X für eine Gruppe *-CH₂-CH(OH)-CH₂-O-(CH₂)₃-* steht,
R¹ und R², jeweils unabhängig voneinander, für eine Hydroxylgruppe stehen,
R³ für ein Wasserstoffatom steht,
n für ganze Zahlen von 1 bis 101 steht,
y für ganze Zahlen von 0 bis 1.000, vorzugsweise von 0 bis 500, insbesondere von 0 bis 100, steht, und
z für ganze Zahlen von 1 bis 1.000, vorzugsweise von 1 bis 500, insbesondere von 1 bis 100, steht,
wobei der hydrolysierte Proteinrest R ein mittleres Molekulargewicht M_{w} von 295 Da bis 26.000 Da aufweist, und
b) mindestens einen Container (C2), enthaltend eine Oxidationsmittelzubereitung, welche in einem kosmetisch verträglichen Träger mindestens ein Oxidationsmittel in einer Gesamtmenge von 0,5 bis 7,0 Gew.-%, bezogen auf das Gesamtgewicht der Oxidationsmittelzubereitung, sowie mindestens eine Säure enthält.

2. Verpackungseinheit nach Anspruch 1, **dadurch gekennzeichnet, dass** das mindestens eine Protein-Siloxan-Polymer pro Aminogruppe des hydrolysierten Proteinrests R 0,1 bis 0,4, insbesondere 0,1 bis 0,3 Moleküle Siloxan enthält.

3. Verpackungseinheit nach einem der vorangehenden Ansprüche, **dadurch gekennzeichnet, dass** das mindestens eine Protein-Siloxan-Polymer 5 bis 30 %, vorzugsweise 5 bis 25 %, bevorzugt 10 bis 30 %, insbesondere 10 bis 15 %, mit Siloxanen umgesetzte Amingruppen und 70 bis 95 %, vorzugsweise 75 bis 95 %, bevorzugt 80 bis 95 %, insbesondere 85 bis 90 %, freie Aminogruppe enthält.

4. Verpackungseinheit nach einem der vorangehenden Ansprüche, **dadurch gekennzeichnet, dass** das mindestens eine Protein-Siloxan-Polymer ein mittleres Molekulargewicht M_{w} von 350 Da bis 90.000 Da, vorzugsweise von 600 bis 80.000 Da, bevorzugt von 1.000 bis 70.000 Da, insbesondere von 2.000 bis 64.000 Da, aufweist.

5. Verpackungseinheit nach einem der vorangehenden Ansprüche, **dadurch gekennzeichnet, dass** das kosmetische Mittel zusätzlich mindestens ein unfunktionalisiertes Proteinhydrolysat, vorzugsweise ein aus der Gattung *Solanum* isoliertes unfunktionalisiertes Proteinhydrolysat, insbesondere ein aus Kartoffel isoliertes unfunktionalisiertes Proteinhydrolysat enthält.

6. Verpackungseinheit nach einem der vorangehenden Ansprüche, **dadurch gekennzeichnet, dass** das Oxidationsmittel in einer Gesamtmenge von 1,0 bis 7,0 Gew.-%, insbesondere von 3,0 bis 7,0 Gew.-%, bezogen auf das Gesamtgewicht der Oxidationsmittelzubereitung, enthalten ist.

## Claims

1. A packaging unit (kit of parts) comprising, packaged separately from one another,
a) at least one container (C1) containing a cosmetic agent for changing the color of keratin fibers, comprising in a cosmetically acceptable carrier
- at least one compound selected from the group of oxidation dye precursors,
- at least one protein-siloxane polymer in a total amount of from 0.02 to 0.2 wt.%, based on the total weight of the cosmetic agent, containing at least one structural unit of formula (I) and/or at least one structural unit of formula (II) 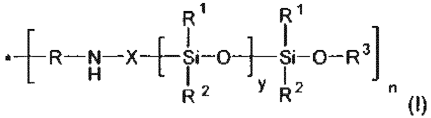 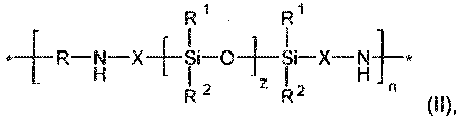 where
R represents a protein hydrolyzate which is isolated from potato and comprises at least one NH2 group,
X represents a *-CH₂-CH(OH)-CH₂-O-(CH₂)₃-* group,
R¹ and R² represent, independently of one another, a hydroxyl group,
R³ represents a hydrogen atom,
n represents integers from 1 to 101,
y represents integers from 0 to 1,000, preferably from 0 to 500, in particular from 0 to 100, and
z represents integers from 1 to 1,000, preferably from 1 to 500, in particular from 1 to 100,
wherein the hydrolyzed protein functional group R has an average molecular weight M_{w} of from 295 Da to 26,000 Da, and
b) at least one container (C2), containing an oxidizing agent preparation which contains in a cosmetically acceptable carrier at least one oxidizing agent in a total amount of from 0.5 to 7.0 wt.%, based on the total weight of the oxidizing agent preparation, and at least one acid.

2. The packaging unit according to claim 1, **characterized in that** the at least one protein-siloxane polymer contains, per amino group of the hydrolyzed protein functional group R, from 0.1 to 0.4, in particular from 0.1 to 0.3, molecules of siloxane.

3. The packaging unit according to either of the preceding claims, **characterized in that** the at least one protein-siloxane polymer contains from 5 to 30%, preferably from 5 to 25%, more preferably from 10 to 30%, in particular from 10 to 15%, of amino groups reacted with siloxanes, and from 70 to 95%, preferably from 75 to 95%, more preferably from 80 to 95%, in particular from 85 to 90%, of a free amino group.

4. The packaging unit according to one of the preceding claims, **characterized in that** the at least one protein-siloxane polymer has an average molecular weight M_{w} of from 350 Da to 90,000 Da, preferably from 600 to 80,000 Da, more preferably from 1,000 to 70,000 Da, in particular from 2,000 to 64,000 Da.

5. The packaging unit according to one of the preceding claims, **characterized in that** the cosmetic agent additionally contains at least one unfunctionalized protein hydrolyzate, preferably an unfunctionalized protein hydrolyzate isolated from the genus *Solanum,* in particular an unfunctionalized protein hydrolyzate isolated from potato.

6. The packaging unit according to one of the preceding claims, **characterized in that** the oxidizing agent is contained in a total amount of from 1.0 to 7.0 wt.%, in particular from 3.0 to 7.0 wt.%, based on the total weight of the oxidizing agent preparation.

## Revendications

1. Unité de conditionnement (Kits-of-Parts) comprenant, fabriqués séparément les uns des autres,
a) au moins un contenant (C1) comprenant un produit cosmétique pour le changement de couleur des fibres kératiniques, compris dans une substance porteuse cosmétique
- au moins une liaison choisie parmi le groupe des précurseurs de coloration par oxydation ;
- au moins un polymère siloxane protéine en une quantité totale comprise entre 0,02 % et 0,2 % en poids par rapport au poids du produit cosmétique comprenant au moins une unité structurelle de forme (I) et/ou au moins une unité structurelle de forme (II) 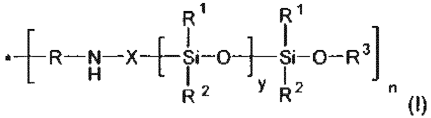 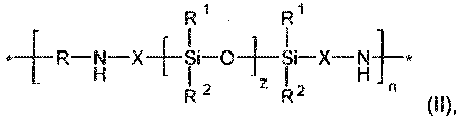 où
R représente un hydrolysat de protéine isolé de la pomme de terre comprenant minimum un groupe NH₂ ;
X représente un groupe *-CH₂-CH(OH)-CH₂-O-(CH₂)₃-* ;
R¹ et R², chacun indépendant l'un de l'autre, représentent un groupe hydroxyle ;
R³ représente un atome d'hydrogène ;
n représente des nombres entiers compris entre 1 et 101 ;
y représente des nombres entiers compris entre 0 et 1 000, de préférence entre 0 et 500, particulièrement entre 0 et 100 ;
z représente des nombres entiers compris entre 1 et 1 000, de préférence entre 1 et 500, particulièrement entre 1 et 100 ;
où le résidu protéique hydrolysé R présente un poids moléculaire moyen M_{w} compris entre 295 Da et 26 000 Da ; et
b) au moins un contenant (C2) comprenant une préparation d'agent d'oxydation qui comprend minimum, dans une substance porteuse cosmétique, un agent d'oxydation en une quantité totale comprise entre 0,5 % et 7,0 % en poids par rapport au poids total de la préparation d'agent d'oxydation ainsi qu'un acide.

2. Unité de conditionnement selon la revendication 1 **caractérisée en ce qu'**elle comprend minimum un polymère siloxane protéine par groupe amine du résidu protéique hydrolysé R 0,1 à 0,4, particulièrement de 0,1 à 0,3 molécule de siloxane.

3. Unité de conditionnement selon les revendications précédentes, **caractérisée en ce qu'**elle comprend minimum un polymère siloxane protéine avec de 5 à 30 %, de préférence de 5 à 25 %, particulièrement de 10 à 30 % et notamment de 10 à 15 % de groupes amine modifiés au siloxane et de 70 à 95 %, de préférence de 75 à 95 %, particulièrement de 80 à 95 % et notamment de 85 à 90 % de groupes amine libres.

4. Unité de conditionnement selon l'une des revendications précédentes, **caractérisée en ce que** minimum un polymère siloxane protéine présente un poids moléculaire M_{w} moyen compris entre 350 et 90 000 Da, de préférence entre 600 et 80 000 Da, particulièrement entre 1 000 et 70 000 Da, notamment entre 2 000 et 64 000.

5. Unité de conditionnement selon l'une des revendications précédentes, **caractérisée en ce que** l'agent cosmétique comprend minimum un hydrolysat de protéine supplémentaire non fonctionnalisé, de préférence un hydrolysat de protéine non fonctionnalisé isolé de la variété Solanum, particulièrement un hydrolysat de protéine non fonctionnalisé isolé de la pomme de terre.

6. Unité de conditionnement selon l'une des revendications précédentes, **caractérisée en ce qu'**il contient un agent d'oxydation en une quantité totale comprise entre 1,0 et 7,0 % en poids, de préférence de 3,0 à 7,0 % en poids par rapport au poids total de la préparation d'agent d'oxydation.
